(19) Europäisches Patentamt
European Patent Office
Office européen des brevets

(11) Numéro de publication: **0 187 095 B1**

# (12) FASCICULE DE BREVET EUROPEEN

(45) Date de publication du fascicule du brevet: **03.05.89**

(51) Int. Cl.⁴: **C 07 K 5/02**, C 07 K 5/08, A 61 K 37/02

(21) Numéro de dépôt: **85402528.5**

(22) Date de dépôt: **18.12.85**

(54) **Esters de tri- et tétrapeptides inhibiteurs de la sécrétion gastrique, procédé d'obtention et compositions pharmaceutiques les contenant.**

(30) Priorité: **20.12.84 FR 8419545**

(43) Date de publication de la demande: **09.07.86 Bulletin 86/28**

(45) Mention de la délivrance du brevet: **03.05.89 Bulletin 89/18**

(84) Etats contractants désignés: **AT BE CH DE FR GB IT LI LU NL SE**

(56) Documents cités:
**FR-A- 2 226 181**
**US-A- 4 012 367**

**JOURNAL OF MEDICINAL CHEMISTRY, vol. 28, no. 3, 1985, pages 273-278, American Chemical Society; J. MARTINEZ et al.: "Structure-activity relationships of C-terminal tri- and tetrapeptide fragments that inhibit gastrin activity"**
**CHEMICAL ABSTRACTS, vol. 94, 1981, page 767, résumé no. 66026k, Columbus, Ohio, US; K. NAKAJIMA et al.: "Synthesis and structure of aziridine-2-carboxylic acid peptides" & PEPT. CHEM. 1976 (PUB. 1977), 14th, 17-20**

(73) Titulaire: **SANOFI, société anonyme, 40, Avenue George V, F-75008 Paris (FR)**
Titulaire: **CENTRE NATIONAL DE LA RECHERCHE SCIENTIFIQUE (CNRS), 15, Quai Anatole France, F-75700 Paris Cedex 07 (FR)**

(72) Inventeur: **Martinez, Jean, rue des Cévennes, F-34720 Caux (FR)**
Inventeur: **Bali, Jean Pierre, 278, rue du Bosquet, F-34980 Saint Gely du Fesc (FR)**
Inventeur: **Magous, Richard, 318, Bd du Général de Gaulle, F-344400 Lunet (FR)**
Inventeur: **Castro, Bertrand, 1'Estaple - Avenue des Costières, F-34130 Saint Aunes (FR)**
Inventeur: **Demarne, Henri, Le Florence avenue Major Flandre, F-34000 Montpellier (FR)**

(74) Mandataire: **Gillard, Marie-Louise et al, Cabinet Beau de Loménie 55, Rue d'Amsterdam, F-75008 Paris (FR)**

**EP 0 187 095 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux peptides inhibiteurs de la sécrétion gastrique. Elle concerne également un procédé pour leur obtention et les compositions pharmaceutiques les contenant.

La gastrine est une hormone gastrointestinale qui possède, à un degré élevé, le pouvoir de stimuler la sécrétion gastrique. Par ailleurs, la pentagastrine et la tétragastrine sont des peptides synthétiques proches de la séquence C-terminale des 5 ou 4 derniers aminoacides de la gastrine et répondent respectivement aux formules:

Boc – Bêta Ala – Trp – Met – Asp – Phe – $NH_2$
et H – Trp – Met – Asp – Phe –$NH_2$
utilisant pour désigner les alpha-aminoacides et les groupes protecteurs les abréviations à 3 lettres recommandées par la Commission de Nomenclature de l'IUPAC – IUB.

Ces composés sont également des stimulants de la sécrétion gastrique.

Selon la présente invention, il a été trouvé que, de façon surprenante, des dérivés peptidiques dérivés de ces séquences, par suppression du phénylalaninamide terminal et estérification convenable de la fonction carboxyle en alpha de l'acide aspartique, deviennent de puissants inhibiteurs de la sécrétion gastrique.

Les composés selon l'invention répondent à la formule générale:

R – $X_1$ – TRP – $X_2$ – L-Asp – $OCH_2$–$CH_2$– 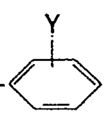 (I)

dans laquelle:

– R représente l'hydrogène ou un groupe protecteur de la fonction amine terminale, tel que t.butoxycarbonyle (Boc), benzyloxycarbonyle (Z) ou alcanoyle inférieur;

– $X_1$ représente soit bêta-Ala, soit une liaison directe entre R et le groupe amine terminal de l'aminoacide TRP;

– TRP désigne soit l'isomère L, soit l'isomère D du tryptophane;

– $X_2$ représente la L-leucine, la L-méthionine ou la L-norleucine;

– Y désigne l'hydrogène, un atome d'halogène (de préférence le fluor ou le chlore), un groupe trifluorométhyle, un groupe cyano ou un groupe nitro.

Les composés selon l'invention peuvent être préparés selon les techniques habituelles de synthèse peptidique soit en phase solide selon Merrifield, soit en phase liquide.

A partir de l'acide aspartique alphaester, on introduit successivement les différents aminoacides présents dans la séquence. Les réactions de couplage sont effectuées soit avec un ester activé de l'acide aminé à introduire, au sein du diméthylformamide et en présence de diisopropyléthylamine et d'hydroxy-1 benzotriazole, soit avec l'acide aminé lui-même, et dans ce cas on opère au sein du diméthylformamide en présence de diisopropyléthylamine et d'hexafluorophosphate de benzotriazolyloxy-tris-diméthylamino-phosphonium (BOP).

Tous les aminoacides sont incorporés sous la forme du dérivé protégé sur l'amine en alpha, le groupe protecteur choisi étant le groupe t-butyloxycarbonyle. Lorsque l'aminoacide utilisé présente dans sa chaîne latérale des fonctions susceptibles de réagir, celles-ci doivent être bloquées au préalable. Ainsi les fonctions acides en bêta de l'acide aspartique doivent être bloquées sous forme d'ester en particulier d'ester benzylique.

Après chaque réaction de couplage, la déprotection de l'amine en alpha est effectuée par hydrolyse acide.

Finalement, les peptides, protégés dans leurs fonctions des chaînes latérales, sont partiellement ou entièrement déprotégés pour conduire aux composés de formule (I).

L'acide aspartique alpha ester de départ est préparé à partir de l'acide aspartique protégé sur l'amine par un groupe tertiobutoxycarbonyle et sur la fonction acide en bêta par un ester benzylique.

L'estérification en alpha peut être effectuée soit par action d'un halogénure de phénéthyle éventuellement substitué, soit par action de l'alcool phénéthylique correspondant en présence de dicyclohexylcarbodiimide et de paradiméthylaminopyridine.

Les exemples suivants, non limitatifs, permettront de mieux comprendre l'invention. Dans ces exemples, on utilisera les abréviations suivantes:

Acides aminés et groupes protecteurs:

| | |
|---|---|
| Asp: | Acide L-aspartique |
| Bêta Ala: | Bêta-alanine |
| Leu: | L-leucine |
| L-Trp: | L-tryptophane |
| D-Trp: | D-tryptophane |
| Boc: | tertiobutyloxycarbonyle |
| OBzl: | ester benzylique |

O-Np: $O_2N$ – 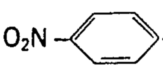 –O–

Autres abréviations:

| | |
|---|---|
| DMF: | diméthylformamide |
| DCC: | dicyclohexylcarbodiimide |
| DIEA: | diisopropyléthylamine |
| TFA: | acide trifluoracétique |
| HOBt: | hydroxy-l benzotriazole |

BOP: 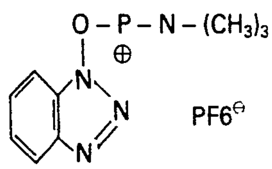 $PF6^{\ominus}$

Exemple 1:

Boc–L–Trp–Leu–Asp–$OCH_2$–$CH_2$– 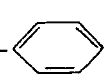

a) Boc–Asp(Bêta OBzl)–$OCH_2$–$CH_2$– 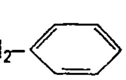

On dissout 4,55 g du sel de césium de l'acide Boc–Asp(Bêta OBzl)–OH dans 100 ml de DMF puis on ajoute 2,22 g de bromure de phénéthyle et agite pendant 12 heures à température ambiante. On concentre sous vide à une température inférieure à 40 °C puis on dissout le résidu dans 250 ml d'acétate d'éthyle. On lave la solution avec une solution saturée de bicarbonate de sodium, avec de l'eau, avec une solution d'acide citrique à 10% puis à nouveau avec de l'eau. On sèche la solution sur sulfate de sodium et évapore le solvant sous vide.

Le résidu cristallise dans un mélange éther-hexane. Rendement 85%; F = 58–60°C; (alpha)D = –20,7° (c = 1,06 DMF).

b) Boc–Leu–Asp(Bêta OBzl)–$OCH_2$–$CH_2$– 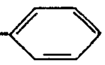

On dissout 4,27 g du produit obtenu ci-dessus dans 20 ml de TFA et laisse ½ heure à température ambiante. On évapore sous vide en ajoutant de l'éther et en maintenant la température inférieure à 40°C. On répète plusieurs fois l'opération. Le résidu huileux est trituré avec de l'éther puis séché sous vide sur potasse.

On dissout 2,32 g du trifluoracétate ainsi obtenu dans 10 ml de DMF avec 1,21 g de Boc-Leu, 2,2 g de BOP et 1,8 ml de DIEA.

On laisse 8 heures à température ambiante puis on concentre sous vide à température inférieure à 40°C. On dissout le résidu dans 200 ml d'acétate d'éthyle et on lave successivement avec une solution saturée de bicarbonate de sodium, avec de l'eau, avec une solution d'acide citrique à 10% et à nouveau avec de l'eau. On sèche la solution sur sulfate de sodium puis concentre sous vide. On chromatographie le résidu sur gel de silice. En éluant avec le mélange acétate d'éthyle-hexane 1/1 vol/vol, on obtient une huile incolore (2,48 g). Rendement 92%; (alpha)D = 19,9° (c = 0,93 DMF).

Chromatographie sur couche mince:

Rf = 0,95 (acétate d'éthyle-hexane 8/2, vol/vol)

Rf = 0,91 (acétone-hexane 7/3 vol/vol).

c) Boc–L–Trp–Leu–Asp(bêta OBzl)–$OCH_2$–

$CH_2$– 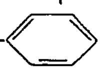

On traite 2 g du produit obtenu en b) par 10 ml de TFA pendant 30 minutes. On évapore plusieurs fois en présence d'éther. On triture le résidu huileux avec de l'éther puis sèche sous vide sur potasse. On dissout le trifluoracétate dans 20 ml de DMF avec 1,7 g de Boc-L Trp-ONp, 0,63 g de HOBt et 1,6 ml de DIEA.

On laisse une nuit à température ambiante puis traite le mélange comme indiqué au paragraphe b).

Par chromatographie sur gel de silice, en éluant avec le mélange hexane-acétate d'éthyle 3/7 vol/vol, on obtient le produit attendu (2,36 g) qui cristallise dans l'éther: F = 135–138°C; (alpha)D = –24° (c = 1,3 DMF): rendement 88%.

d) Boc–L–Trp–Leu–Asp–$OCH_2$–$CH_2$– 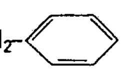

On dissout 0,73 g du produit obtenu en c) dans 50 ml d'éthanol à 95° et on hydrogène, à température et pression ordinaires, la solution en présence de 0,07 g de palladium sur charbon à 10%.

Après 4 heures la réaction est terminée. On filtre le catalyseur et concentre sous vide à température inférieure à 40°C.

Le résidu trituré avec un mélange éther-hexane donne une poudre incolore (0,483 g): F = 95–100°C; (alpha)D = –22,7° (c = 1,3 DMF); rendement 76%.

Exemple 2:

Boc–bêta Ala–L–Trp–Leu–Asp–$OCH_2$–$CH_2$– 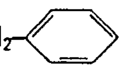

a) Boc–bêta Ala–L–Trp–Leu–Asp(bêta OBzl)–

$OCH_2$–$CH_2$– 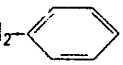

On traite 0,73 g du produit obtenu à l'exemple 1c) par 3 ml de TFA comme indiqué précédemment. Après séchage, le trifluoracétate ainsi obtenu est dissous dans 10 ml de DMF avec 0,189 g de Boc-bêta Ala, 0,398 g de BOP et 0,4 ml de DIEA.

On laisse une nuit à température ambiante et traite comme à l'exemple 1b). Par chromatographie sur gel de silice, en éluant par le mélange acétate d'éthyle-hexane 7/3 vol/vol, on obtient le produit attendu (0,595 g) qui cristallise par trituration avec de l'éther: F = 163–166°C; (alpha)D = –18,9° (c = 1,2 DMF); rendement 83%.

b) Boc–bêta Ala–L–Trp–Leu–Asp–$OCH_2$–

$CH_2$– 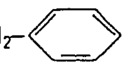

On hydrogène 0,398 g du produit obtenu ci-dessus selon le mode opératoire de l'exemple 1d). De la même façon, on isole une poudre incolore: F = 100–103°C; (alpha)D = –17,7° (c = 1,7 DMF); rendement 79%.

Exemple 3:

Boc–D–Trp–Leu–Asp–$OCH_2$–$CH_2$– 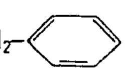

a) Boc–D–Trp–Leu–Asp(bêta OBzl)–$OCH_2$–

$CH_2$– 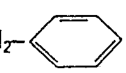

On dissout 1,36 g de trifluoracétate de

H–Leu–Asp(bêta OBzl)–O–$CH_2$–$CH_2$– 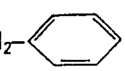

(préparé selon l'exemple 1) dans 20 ml de DMF avec 1,06 g de BOP, 0,76 g de Boc-D-Trp et 0,86 ml de DIEA.

Après 8 heures à température ambiante, on traite comme indiqué à l'exemple 1b). On chromatographie sur gel de silice et, par élution avec un mélange acétate d'éthyle-hexane 5/5 vol/vol, on isole le produit attendu qui cristallise par trituration avec un mélange éther-hexane: F = 67–70°C; (alpha)D= –14,1° (c = 1 DMF); rendement 91%.

b) Boc–D–Trp–Leu–Asp–OCH$_2$–CH$_2$–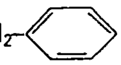

On hydrogène 0,242 g du produit obtenu ci-dessus comme indiqué à l'exemple 1d). On isole de la même façon une poudre incolore: F = 108–110°C; (alpha)D = 15,8° (c = 1 DMF); rendement 78%.

Exemple 4:

Boc–bêta Ala–D–Trp–Leu–Asp–OCH$_2$–CH$_2$–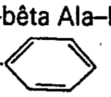

a) Boc–bêta Ala–D–Trp–Asp(bêta OBzl)–OCH$_2$–CH$_2$–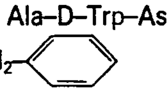

A partir du peptide protégé de l'exemple 3a), on opère comme dans l'exemple 2a). Par le même traitement, et après chromatographie sur gel de silice en éluant avec le mélange acétate d'éthyle-hexane 1/1 vol/vol, on obtient une poudre incolore: F = 153–156°C; (alpha)D = –27,8° (c = 1,4 DMF); rendement 79%.

b) Boc–bêta Ala–D–Trp–Leu–Asp–OCH$_2$–CH$_2$–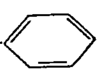

Selon le procédé à l'exemple 1d), on hydrogène le produit précédent. On obtient une poudre incolore: F = 105–110°C; (alpha)D = –20,4° (c = 1,2 DMF); rendement 84%.

Exemple 5:

Boc–L–Trp–Leu–Asp–OCH$_2$—CH$_2$–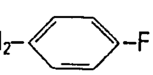–F

a) Boc–Asp(bêta OBzl)–OCH$_2$–CH$_2$–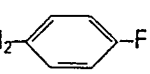–F

On dissout 2,2 g de Boc-Asp(bêta OBzl)-OH dans 70 ml de dichlorométhane avec 0,924 g de para-fluorophénéthyl alcool, 1,24 g de DCC et 0,732 g de diméthylamino-4 pyridine. On laisse une nuit à température ambiante puis on filtre la dicyclohexylurée et concentre sous vide.

On reprend le résidu dans l'acétate d'éthyle. On lave la solution successivement avec une solution saturée de bicarbonate de sodium, avec de l'eau, avec une solution d'acide citrique à 10% et à nouveau avec de l'eau. On sèche la solution sur sulfate de sodium et évapore le solvant sous vide. On chromatographie le résidu sur colonne de gel de silice. En éluant avec un mélange hexane-acétate d'éthyle 8/2 vol/vol, on obtient une huile incolore (1,82 g): (alpha)D = –16° (c = 1,9 DMF); rendement 68%.

Chromatographie sur couche mince:
Rf = 0,64 (chloroforme)
Rf = 0,85 (acétate d'éthyle-hexane 1/1 vol/vol)

b) Boc–Leu–Asp(bêta OBzl)–OCH$_2$–CH$_2$–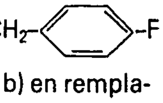–F

On opère comme dans l'exemple 1b) en remplaçant

Boc–Asp(bêta OBzl)–OCH$_2$–CH$_2$–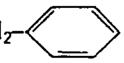

par une quantité + Bz équivalente de

Boc–Asp(bêta OBzl)–OCH$_2$–CH$_2$–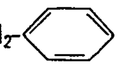–F.

On traite de la même façon, et, après chromatographie sur gel de silice en éluant avec le mélange hexane-acétate d'éthyle 8/2 vol/vol, on obtient avec un rendement de 89% une huile incolore: (alpha)D = –18,5° (c = 0,9 DMF).

Chromatographie sur couche mince:
Rf = 0,66 (chloroforme)
Rf V 0,59 (acétate d'éthyle-hexane 1/1 vol/vol).

c) Boc–L–Trp–Leu–Asp(bêta OBzl)–OCH$_2$–CH$_2$–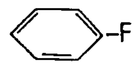–F

On opère comme dans l'exemple 1c) à partir du produit obtenu ci-dessus. On traite de la même façon puis chromatographie sur gel de silice pour obtenir un produit cristallisé: F = 90–92°C; (alpha)D = –23° (c = 1,2 DMF); rendement 77%.

d) Boc–L–Trp–Leu–Asp–OCH$_2$–CH$_2$–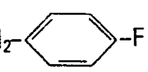–F

Comme indiqué précédemment, on hydrogène en présence de palladium sur charbon le produit obtenu ci-dessus. Par le même traitement, on obtient un solide: F = 103–107°C; (alpha)D = –19° (c = 1 DMF); rendement 72%.

Exemple 6:

Boc–bêta Ala–L–Trp–Leu–Asp–OCH$_2$–CH$_2$–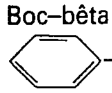–F

a) Boc–bêta Ala–L–Trp–Leu–Asp(bêta OBzl)–OCH$_2$–CH$_2$–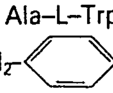–F

On opère comme dans l'exemple 2a) à partir du peptide protégé de l'exemple 5c). De la même façon, on obtient le produit attendu: F = 80–85°C (décomposition).

Chromatographie sur couche mince:
Rf = 0,5 (acétate d'éthyle-hexane 7/3 vol/vol).

b) Boc–bêta Ala–L–Trp–Leu–Asp–OCH$_2$–CH$_2$–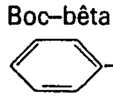–F

On déprotège le peptide obtenu ci-dessus par hydrogénation catalytique comme indiqué dans l'exemple 1d).

On obtient un solide: F = 110–115°C (décomposition).

Chromatographie sur couche mince:
Rf = 0,82 (acétate d'éthyle, pyridine, acide acétique, eau 80/20/3/3 vol/vol).

Exemple 7:

Boc–L–Trp–Leu–Asp–$OCH_2$–$CH_2$– 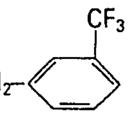

a) Boc–Asp(bêta OBzl)–$OCH_2$–$CH_2$– 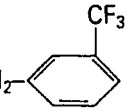

On opère comme dans l'exemple 5a) en remplaçant le parafluorophénéthyl-alcool par une quantité équivalente de métatrifluorométhylphénéthyl-alcool.

Après chromatographie sur gel de silice en éluant avec le mélange hexane-acétate d'éthyle 2/8 vol/vol, on obtient une huile: (alpha)D = −12,9° (c = 0,8 DMF); rendement 71%.

Chromatographie sur couche mince:
Rf = 0,67 (chloroforme)
Rf = 0,69 (acétate d'éthyle-hexane 1/1 vol/vol).

b) Boc–Leu–Asp(bêta OBzl)–$OCH_2$–$CH_2$– 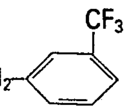

A partir de l'ester préparé ci-dessus, on opère comme dans l'exemple 1b). Après chromatographie sur colonne de gel de silice en éluant avec le mélange acétate d'éthyle-hexane 1/1 vol/vol, on obtient un produit cristallisé: F = 72–75°C; (alpha)D = −18° (c = 1,1 DMF); rendement 78%.

c) Boc–L–Trp–Leu–Asp(bêta OBzl)–$OCH_2$–

$CH_2$– 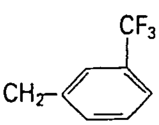

A partir du produit obtenu en b), on opère comme dans l'exemple 1c).

Après chromatographie sur colonne de gel de silice en éluant avec le mélange acétate d'éthyle-hexane 1/1 vol/vol, on obtient une poudre incolore; F = 55–60°C; (alpha)D = −5,6° (c = 1,9 DMF); rendement 81%.

d) Boc–L–Trp–Leu–Asp–$OCH_2$–$CH_2$– 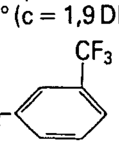

On déprotège le produit obtenu au paragraphe c) par hydrogénation catalytique comme indiqué à l'exemple 1d).

On obtient dans un mélange éther-hexane, un solide: F = 95–100°C; (alpha)D = −19,6° (c = 0,9 DMF); rendement 79%.

Les composés selon l'invention ont été étudiés en ce qui concerne leurs propriétés thérapeutiques. Plus particulièrement, ces composés ont été étudiés in vivo en ce qui concerne leur effet sécrétoire gastrique chez le rat.

Le modèle choisi pour la mesure de l'effet sécrétoire est celui de l'estomac de rat anesthésié reperfusé. Le protocole suivi est une modification de celui décrit préalablement par Ghosh et Schild.

Un rat mâle de souche Wistar de 300 g, à jeun de 18 h, est anesthésié à l'uréthane (solution à 10%, 1,5 ml/100 g, i.p.). On pratique ensuite une trachéotomie et un cathétérisme de la veine du pénis qui permettra l'administration i.v. des peptides. On place ensuite une canule dans l'œsophage jusqu'au cardia et une seconde dans le duodénum (par une duodénotomie effectuée à 3 cm environ du pylore) jusque dans la zone antrale gastrique.

Un soluté propionique-succinique (pH 5,5), qui donne une variation linéaire du pH en fonction de la concentration en ions H+, est utilisé pour perfuser l'estomac en circuit ouvert ou fermé avec un débit de 3 ml/min. La température corporelle ainsi que celle du soluté sont contrôlées et maintenues à 30°C. La sécrétion acide de l'estomac va entraîner une variation de pH détectée par l'électrode de verre et enregistrée en fonction du temps. Après stabilisation de la sécrétion basale, on injecte la gastrine par voie intraveineuse soit en perfusion, soit en injection unique. La réponse est enregistrée en fonction du temps et la quantité d'acide sécrété est mesurée sur l'enregistrement par différence avec la sécrétion basale.

La même expérience est réalisée soit en injectant le peptide à étudier i.v. sur un plateau de sécrétion acide stimulée par la gastrine, soit en associant le peptide avec le stimulant dans des rapports de concentration variables.

En faisant varier la dose du produit à étudier et en mesurant les effets correspondants, il est possible pour chaque produit de déterminer la Dose Efficace 50% ou dose qui inhibe de 50% la sécrétion gastrique induite par la gastrine.

Les résultats obtenus avec différents produits de l'invention sont les suivants:

| Peptide | DE 50 (mg/kg) |
|---|---|
| Exemple 1 | 0,1 |
| Exemple 2 | 0,04 |
| Exemple 4 | 0,2 |
| Exemple 5 | 0,08 |
| Exemple 6 | 0,02 |
| Exemple 7 | 0,5 |

Ces résultats montrent que les composés selon l'invention présentent un effet inhibiteur très important sur la sécrétion gastrique. Par ailleurs, ces composés sont peu toxiques.

Par suite, les composés selon l'invention pourront être utilisés en thérapeutique humaine dans tous les cas où il est utile de diminuer la sécrétion gastrique et en particulier pour le traitement des ulcères gastro-duodénaux.

Les composés de la présente invention peuvent être utilisés de préférence par voie injectable, intraveineuse, intramusculaire ou sous-cutanée. Ils sont utilisés dans un solvant tel que le sérum physiologique (solution saline isotonique).

La posologie peut varier suivant l'intensité de l'effet thérapeutique recherchée, la gravité de l'affection à traiter et la voie d'administration utilisée. Elle doit donc être déterminée pour chaque patient en fonction de ces divers critères. Le plus souvent elle est comprise entre 0,1 et 10 mg de principe actif par kg de poids corporel.

L'invention concerne donc également les compositions pharmaceutiques contenant à titre d'ingrédient actif un peptide selon l'invention en combinaison avec un véhicule pharmaceutiquement acceptable, tel que le sérum physiologique.

**Revendications pour les Etats contractants: BE, CH, DE, FR, GB, IT, LI, LU, NL, SE**

1. A titre de nouveaux produits, les peptides de formule:

R – $X_1$ – TRP – $X_2$ – L-Asp –$OCH_2$–$CH_2$– 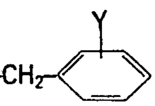 (I)

dans laquelle:

– R représente l'hydrogène ou un groupe protecteur de la fonction amine terminale, tel que t.butoxycarbonyle (Boc), benzyloxycarbonyle (Z) ou alcanoyle inférieur;

– $X_1$ représente soit bêta Ala, soit une liaison directe entre R et le groupe amine terminal de l'amino acide TRP;

– TRP désigne soit l'isomère L, soit l'isomère D du tryptophane;

– $X_2$ représente la L-leucine, la L-méthionine ou la L-norleucine;

– Y désigne l'hydrogène, un atome d'halogène (de préférence le fluor ou le chlore), un groupe trifluorométhyle, un groupe cyano ou un groupe nitro.

2. Peptides selon la revendication 1, caractérisés en ce qu'ils sont l'un des peptides ci-après:

Boc–L–Trp–Leu–Asp–$OCH_2$–$CH_2$– 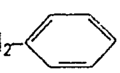

Boc–bêta Ala–L–Trp–Leu–Asp–$OCH_2$–$CH_2$– 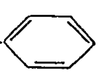

Boc–D–Trp–Leu–Asp–$OCH_2$–$CH_2$– 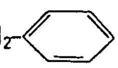

Boc–bêta Ala–D–Trp–Leu–Asp–$OCH_2$–$CH_2$– 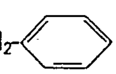

Boc–L–Trp–Leu–Asp–$OCH_2$–$CH_2$– 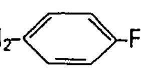

Boc–bêta Ala–L–Trp–Leu–Asp–$OCH_2$–$CH_2$–

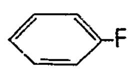

Boc–L–Trp–Leu–Asp–$OCH_2$–$CH_2$– 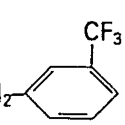

ou les peptides déprotégés correspondants.

3. Procédé pour l'obtention des peptides selon l'une des revendications 1 ou 2, caractérisé en ce qu'il consiste, à partir de l'acide aspartique α-ester, à introduire successivement les différents aminoacides de la séquence par synthèse peptidique en phase solide ou en phase liquide.

4. Composition pharmaceutique inhibitrice de la sécrétion gastrique, caractérisée en ce qu'elle contient à titre d'ingrédient actif un peptide selon l'une des revendications 1 ou 2, en association avec un véhicule pharmaceutiquement acceptable.

**Revendications pour l'Etat contractant: AT**

1. Procédé pour l'obtention des peptides de formule:

R – $X_1$ – TRP – $X_2$ – L-Asp – $OCH_2$–$CH_2$– 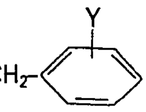 (I)

dans laquelle:

– R représente l'hydrogène ou un groupe protecteur de la fonction amine terminale, tel que t.butoxycarbonyle (Boc), benzyloxycarbonyle (Z) ou alcanoyle inférieur;

– $X_1$ représente soit bêta Ala, soit une liaison directe entre R et le groupe amine terminal de l'amino acide TRP;

– TRP désigne soit l'isomère L, soit l'isomère D du tryptophane;

– $X_2$ représente la L-leucine, la L-méthionine ou la L-norleucine;

– Y désigne l'hydrogène, un atome d'halogène (de préférence le fluor ou le chlore), un groupe trifluorométhyle, un groupe cyano ou un groupe nitro,

caractérisé en ce qu'il consiste, à partir de l'acide aspartique α-ester, à introduire successivement les différents aminoacides de la séquence par synthèse peptidique en phase solide ou en phase liquide.

2. Procédé selon la revendication 1, caractérisé en ce que R est le groupe t.butoxycarbonyle (Boc), $X_1$ est bêta Ala ou une liaison directe et Y est l'hydrogène, le groupe fluoro ou le groupe trifluorométhyle.

3. Utilisation des peptides obtenus par le procédé selon l'une des revendications 1 ou 2, pour la préparation de compositions pharmaceutiques contenant à titre d'ingrédient actif un peptide de formule I en combinaison avec un véhicule pharmaceutiquement acceptable.

**Patentansprüche für die Vertragsstaaten: BE, CH, DE, FR, GB, IT, LI, LU, NL, SE**

1. Als neue Produkte die Peptide der Formel

R – $X_1$ – TRP – $X_2$ – L-Asp – $OCH_2$–$CH_2$– 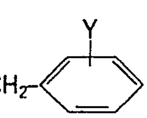 (I)

worin,

– R Wasserstoff oder eine Schutzgruppe der terminalen Aminofunktion, wie tert. Butoxycarbonyl (Boc), Benzyloxycarbonyl (Z) oder Niedrigalkanoyl, darstellt;

– $X_1$ entweder für Beta-Ala oder eine Direktbindung zwischen R und der terminalen Aminogruppe der Aminosäure TRP steht;

– TRP entweder das L-Isomere oder das D-Isomere von Tryptophan bezeichnet;

– $X_2$ für L-Leucin, L-Methionin oder L-Norleucin steht;

– Y Wasserstoff, ein Halogenatom (vorzugsweise Fluor oder Chlor), eine Trifluormethylgruppe, eine Cyanogruppe oder eine Nitrogruppe bezeichnet.

2. Peptide nach Anspruch 1, dadurch gekennzeichnet, daß sie eines der nachstehenden Peptide:

Boc–L–Trp–Leu–Asp–$OCH_2$–$CH_2$– 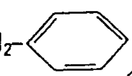

Boc–bêta Ala–Trp–Leu–Asp–$OCH_2$–$CH_2$– 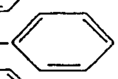

Boc–D–Trp–Leu–Asp–$OCH_2$–$CH_2$– 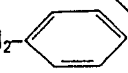

Boc–bêta Ala–D–Trp–Leu–Asp–$OCH_2$–$CH_2$– 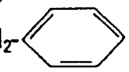

Boc–L–Trp–Leu–Asp–$OCH_2$–$CH_2$– 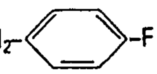

Boc–bêta Ala–L–Trp–Leu–Asp–$OCH_2$–$CH_2$–

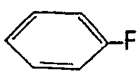

Boc–L–Trp–Leu–Asp–$OCH_2$–$CH_2$– 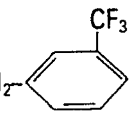

oder die entsprechenden entschützten Peptide sind.

3. Verfahren zur Darstellung der Peptide nach einem der Ansprüche 1 oder 2, dadurch gekennzeichnet, daß es darin besteht, daß, ausgehend vom Asparaginsäure-α-ester, nacheinander die verschiedenen Aminosäuren der Sequenz durch Peptidsynthese in fester Phase oder in flüssiger Phase eingebracht werden.

4. Pharmazeutische Zusammensetzung zur Inhibition der Magensaftsekretion, dadurch gekennzeichnet, daß sie als aktives Ingrediens ein Peptid nach einem der Ansprüche 1 oder 2 in Kombination mit einem pharmazeutisch akzeptablen Vehikel enthält.

**Patentansprüche für den Vertragsstaat: AT**

1. Verfahren zur Darstellung der Peptide der Formel:

R – $X_1$ – TRP – $X_2$ – L-Asp – $OCH_2$–$CH_2$– 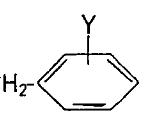 (I)

worin:

– R Wasserstoff oder eine Schutzgruppe der terminalen Aminofunktion, wie tert. Butoxycarbonyl (Boc), Benzyloxycarbonyl (Z) oder Niedrigalkanoyl, darstellt;

– $X_1$ entweder für Beta-Ala oder eine Direktbindung zwischen R und der terminalen Aminogruppe der Aminosäure TRP steht;

– TRP entweder das L-Isomere oder das D-Isomere von Tryptophan bezeichnet;

– $X_2$ für L-Leucin, L-Methionin oder L-Norleucin steht;

– Y Wasserstoff, ein Halogenatom (vorzugsweise Fluor oder Chlor), eine Trifluormethylgruppe, eine Cyanogruppe oder eine Nitrogruppe bezeichnet, dadurch gekennzeichnet, daß es darin besteht, daß, ausgehend vom Asparaginsäure-α-ester, nacheinander die verschiedenen Aminosäuren der Sequenz durch Peptidsynthese in fester Phase oder in flüssiger Phase eingebracht werden.

2. Verfahren nach Anspruch 1, dadurch gekennzeichnet, daß R die Gruppe tert. Butoxycarbonyl (Boc) ist, $X_1$ für Beta-Ala oder eine Direktbindung steht und Y Wasserstoff, Fluor oder Trifluormethyl ist.

3. Verwendung der durch das Verfahren nach einem der Ansprüche 1 oder 2 erhaltenen Peptide zur Herstellung von pharmazeutischen Zusammensetzungen, die als aktives Ingrediens ein Peptid der Formel I in Kombination mit einem pharmazeutisch akzeptablen Vehikel enthalten.

**Claims for the contracting states: BE, CH, DE, FR, GB, IT, LI, LU, NL? SE**

1. As new products, the peptides of the formula:

R–$X_1$–TRP–$X_2$–L-Asp–$OCH_2$–$CH_2$– 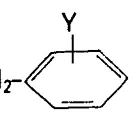 (I)

in which:

– R represents hydrogen or a protecting group for the terminal amine function, such as t-butoxycarbonyl (Boc), benzyloxycarbonyl (Z) or lower alkanoyl;

– $X_1$ represents either beta-Ala or a direct bond between R and the terminal amine group of the amino acid TRP;

– TRP denotes either the L isomer or the D isomer of tryptophan;

– $X_2$ represents L-leucine, L-methionine or L-norleucine;

– Y denotes hydrogen, a halogen atom (preferably fluorine or chlorine), a trifluoromethyl group, a cyano group or a nitro group.

2. Peptides according to claim 1, characterized in that they are one of the following peptides:

Boc–L–Trp–Leu–Asp–$OCH_2$–$CH_2$– 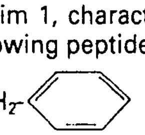

Boc–beta Ala–L–Trp–Leu–Asp–$OCH_2$–$CH_2$– 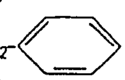

Boc–D–Trp–Leu–Asp–$OCH_2$–$CH_2$– 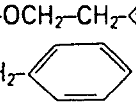

Boc–beta Ala–D–Trp–Leu–Asp–$OCH_2$–$CH_2$– 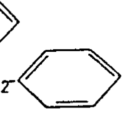

Boc–L–Trp–Leu–Asp–$OCH_2$–$CH_2$– 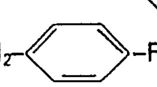

Boc–beta Ala–L–Trp–Leu–Asp–$OCH_2$–$CH_2$–

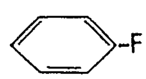

Boc–L–Trp–Leu–Asp–$OCH_2$–$CH_2$– 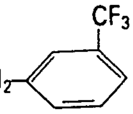

or the corresponding deprotected peptide.

3. Process for the preparation of the peptides according to one of claims 1 or 2, characterized in that, starting from the α-ester of aspartic acid, said process consists in introducing the different amino acids of the sequence in sucession by peptide synthesis in solid phase or in liquid phase.

4. Pharmaceutical composition for inhibiting gastric secretion, characterized in that it contains a

peptide as claimed in one of claims 1 or 2 as the active ingredient, in association with a pharmaceutically acceptable vehicle.

**Claims for the contracting state: AT**

1. Process for the preparation of peptides of formula:

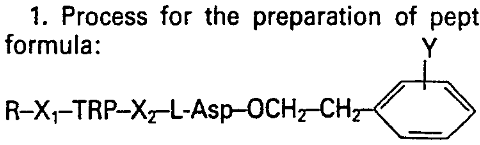

(I)

in which:

– R represents hydrogen or a protecting group for the terminal amine function, such as t-butoxycarbonyl (Boc), benzyloxycarbonyl (Z) or lower alkanoyl;

– $X_1$ represents either beta-Ala or a direct bond between R and the terminal amine group of the amino acid TRP;

– TRP denotes either the L isomer or the D isomer of tryptophan;

– $X_2$ represents L-leucine, L-methionine or L-norleucine;

– Y denotes hydrogen, a halogen atom (preferably fluorine or chlorine), a trifluoromethyl group, a cyano group or a nitro group,

characterized in that, starting from the α-ester of aspartic acid, said process consists in introducing the different amino acids of the sequence in succession by peptide synthesis in solid phase or in liquid phase.

2. Process according to claim 1, characterized in that R is the t-butoxycarbonyl (Boc), $X_1$ is beta-Ala or a direct bond and Y is hydrogen, the fluoro group or the trifluoromethyl group.

3. Use of the peptides obtained by the process according to one of claims 1 or 2, in the preparation of pharmaceutical compositions containing a peptide of formula I as the active ingredient, in association with a pharmaceutically acceptable vehicle.